# EUROPÄISCHE PATENTSCHRIFT

(11) **EP 0 904 791 B1**
(45) Veröffentlichungstag und Bekanntmachung des Hinweises auf die Patenterteilung: **03.12.2003**
(21) Anmeldenummer: 98118086.2
(22) Anmeldetag: 24.09.1998
(51) Int. Cl.: A61M 15/00

(54) **Inhalationsvorrichtung**
Inhalation device
Dispositif pour inhalation

(30) Priorität: 24.09.1997 DE 19742206
(43) Veröffentlichungstag der Anmeldung: 31.03.1999
(73) Patentinhaber: PARI GmbH Spezialisten für effektive Inhalation, 82319 Starnberg (DE)
(72) Erfinder: Ott, Oskar, 82327 Tutzing/Kampberg (DE); Ruf, Hartwig, 82377 Penzberg (DE); Knoch, Martin, Dr., 82335 Berg (DE)
(74) Vertreter: Zangs, Rainer E., Dipl.-Ing.

(56) Entgegenhaltungen:
- EP-A- 0 326 174
- EP-A- 0 438 862
- US-A- 4 819 625
- US-A- 4 952 903
- US-A- 5 408 574

## Beschreibung

Die vorliegende Erfindung betrifft eine Inhalationsvorrichtung mit einem Behälter zur Aufnahme einer Flüssigkeit und einem elektrischen Heizelement, das in wärmeleitendem Kontakt mit dem Behälter steht, zum Erwärmen einer in dem Behälter aufgenommenen Flüssigkeit.

Zur Behandlung von Atemwegserkrankungen werden verschiedene Inhalationsapparate, wie beispielsweise Vernebler, Zerstäuber und Inhalatoren verwendet. Mittels derartiger Apparate werden therapeutische Wirkstoffe in fein verteilter Form von einem Patienten eingeatmet. In der Regel ist eine Erwärmung der meist in flüssiger bzw. gelöster Form vorliegenden Wirkstoffe vorteilhaft.

Aus DE 30 43 537 ist ein Zerstäuber bekannt, der für die Verneblung von flüssigen oder festen Stoffen für Inhalationszwecke vorgesehen ist. Der bekannte Zerstäuber weist einen Behälter und ein separates elektrisches Heizelement auf, das in wärmeleitendem Kontakt mit dem Behälter steht. Als Heizelement dient hierbei ein PCT-Thermistor, der in einem Körper hoher Wärmeleitfähigkeit angeordnet ist. Dieser Körper ist typischerweise ein Metallblock, und umgibt teilweise den Behälter. Dadurch wird eine gute thermische Kopplung zwischen Heizelement und Behälter gewährleistet, und das Heizen des Behälters erfolgt direkt über eine Erwärmung des Metallblocks.

Ein damit ausgestatteter Vernebler weist allerdings große Abmessungen auf, da der für den Wärmeübertrag von Heizelement auf Behälter notwendige Metallblock viel Platz beansprucht. Das gesamte Gerät ist somit globig und unhandlich, und kann nur als Tischgerät verwendet werden.

Ein weiterer Nachteil ist die hohe Heizleistung, die benötigt wird um die Temperatur des Metallblocks, und somit die Temperatur der sich in dem Behälter befindende Flüssigkeit, über den PCT-Thermistor zu steuern. Die Temperatursteuerung bzw. -regelung ist außerdem äußerst träge, da der gesamte Metallblock erwärmt werden muß bzw. die Abkühlung des Metallblocks sehr langsam erfolgt.

Der vorliegenden Erfindung liegt daher das Problem zugrunde, eine Inhalationsvorrichtung bereitzustellen, die wesentlich kleinere Abmessungen als bisher bekannte Vorrichtungen aufweist, und somit besonders anwenderfreundlich ist.

Dieses Problem wird erfindungsgemäß durch die im Patentanspruch 1 beschriebene Vorrichtung gelöst. Vorteilhafte Ausgestaltungen ergeben sich aus den Unteransprüchen.

Ein zentraler Gedanke der Erfindung besteht hierbei darin, daß der Behälter aus einer Keramik mit einer Wärmeleitfähigkeit λ > 15 W/Km besteht, und daß das Heizelement in Form eines Dickschichtwiderstands unmittelbar auf der Außenseite des Behälterbodens angeordet ist.

Durch eine derartige Ausgestallung erübrigt sich ein großer kolbiger Metallblock, und die Abmessungen der erfindungsgemäßen Inhalationsvorrichtung reduziert sich erheblich. Dies hat zur Folge, das die erfindungsgemäße Vorrichtung kein Tischgerät mehr sein muß sondern während des Inhalationsvorgangs von dem Patienten gehalten werden kann. Dies stellt eine konfortablere Anwendung für den Patienten dar, da er sich beim Inhaliervorgang nicht in gerümmter Haltung über ein Tischgerät beugen muß. Statt dessen kann er eine bequeme Sitzhaltung einnehmen, und das Inhalationsgerät in einer für ihn günstigen Stellung halten.

Im folgenden wird die Erfindung anhand eines Ausführungsbeispiels unter Bezugnahme auf die begleitenden Abbildungen genauer beschrieben. Darin zeigt
- Fig. 1: eine Schnittansicht des in der erfindungsgemäßen Inhalationsvorrichtung verwendeten Behälters;
- Fig. 2: eine Bodenansicht des in Fig. 1 gezeigten Behälters, die die Anordnung des Heizelements auf der Aussenbodenfläche des Behälters zeigt;
- Fig. 3: eine Schnittansicht des in Fig. 1 gezeigten Behälters, zusammen mit einem Isolierkörper und Anschlüssen zur Regelung des Heizelements;
- Fig. 4: eine perspektivische Darstellung der in Fig. 3 gezeigten Anordnung;
- Fig. 5: eine weitere perspektivische Darstellung der in Fig. 3 gezeigten Anordnung.

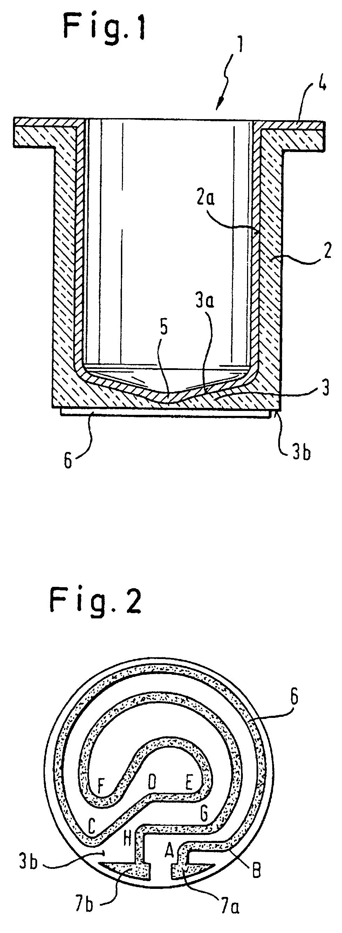

Fig. 1 zeigt eine Schnittansicht des in der erfindungsgemäßen Inhalationsvorrichtung verwendeten Behälters 1. Der Behälter 1 hat eine zylindrische Seitenwand 2 mit konstanter Wandstärke und ist an einem Ende mit einem kreisförmigen Boden 3 abgeschlossen. Die Innenseite 3a des Bodens 3 ist so ausgebildet, daß sie' konisch auf den Mittelbereich 5 des Bodens 3 zuläuft. Somit nimmt die Dicke des Bodens 3 zur Mitte hin ab. Durch die in Richtung Mittelbereich 5 zulaufende innere Bodenfläche 3a fließt die sich in dem Behälter befindende Flüssigkeit selbsttätig in die Mitte des Behälterbodens. Eine derartige Ausgestaltung ist speziell bei einer Verwendung des Behälters in Verneblern vorteilhaft, da hier über eine Zerstäuberdüse, die senkrecht zum Mittelbereich 5 angeordnet ist, und die in die sich in dem Behälter befindenden Flüssigkeit eintaucht, die Flüssigkeit aus dem Becher angesaugt wird.

Auf der äußeren Oberfläche 3b des Bodens 3 ist ein Heizelement 6 in Form eines Dickschichtwiderstands angeordnet. Der Dickschichtwiderstand wird mittels Siebdruckverfahren auf die polierte und plan geschliffene äußere Bodenfläche 3b des Behälters 1 aufgedruckt und anschließend eingebrannt. Der Widerstand des Dickschitwiderstands beträgt hierbei typischerweise ca. 12 Ohm. Durch eine derartige Anordnung des Heizelements wird ein optimaler thermischer Kontakt zwischen Heizelement und der sich in dem Behälter befindenden Flüssigkeit erreicht, da sich das Heizelement unmittelbar an dem Behälterbodens, bzw. sich so nahe wie möglich an der zu erwärmenden Flüssigkeit befindet, ohne in direkten Kontakt mit der Flüssigkeit zu treten. Da der Boden 3 des Behälters 1 sehr dünn ausgebildet werden kann, findet überdies ein rascher Übergang der Wärme statt.

Die Ausgestalltung des Heizelements 6 auf der Aussenbodenfläche 3b des Behälters 1 geht aus Fig. 2 hervor. Die beiden Enden des Heizelements sind als Anschlüsse 7a und 7b an den äußeren Rand der Aussenbodenfläche 3b herausgeführt. Das Heizelement 6 erstreckt sich hierbei geradlinig vom Anschlußelement 7a in Richtung Mittelbereich der Bodenfläche, bishin zu dem in Fig. 2 mit A bezeichneten Punkt. Das Heizelement 6 knickt hier in einem Winkel von 90° in Richtung des Bodenrands ab, und erstreckt sich bis zu dem in Fig. 2 mit B bezeichneten Punkt. Von Punkt B bis Punkt C beschreibt das Heizelement eine kreisförmige Bahn, entgegen Uhrzeigersinn entlang des Rands der Aussenbodenfläche verlaufend, und überstreicht bishin zu Punkt C einen Winkel von ungefähr 320°. An Punkt C knickt das Heizelement unter einem Winkel von ungefähr 45° in Richtung Mittelbereich 5 ab, und verläuft dann von Punkt D bis Punkt E parallel zu dem von Punkt A bis Punkt B verlaufenden geraden Bereich des Heizelements. Von Punkt E an überstreicht das Heizelement kreisförmig, den Mittelbereich 5 umgebend, einen Winkel von ungefähr 270°. In Punkt F dreht sich der Drehsinn des Verlaufs des Heizelements um (nun im Uhrzeigersinn) und überstreicht bis Punkt G einen Winkelbereich von ungefähr 300°. In Punkt G knickt das Heizelement ab, so daß der Bereich von G bis H parallel zu dem Bereich A bis B bzw. E bis D verläuft. In Punkt H knickt das Heizelement dann um 90° in Richtung der zweiten Anschlußfläche 7b ab, um mit dieser in Verbindung zu treten. Ein derart ausgebildetes Heizelement bewirkt eine gleichmäßige Verteilung der Heizleistung auf die sich in dem Behälter befindenden Flüssigkeit. Zudem sei erwähnt, daß der Mittelbereich 5 nicht von dem Heizelement bedeckt ist, da dieser Bereich der Bodenfläche, wie zuvor erwähnt, eine reduzierte Dicke hat und somit empfindlicher als der verbleibende Bereich des Bodens 3 ist. Durch Aussparen des Mittelbereichs 5 wird ein Überheizen des dünnen Bodenbereichs vermieden.

Die Anordnung des Heizelements ist jedoch nicht auf die oben beschriebene Anordung beschränkt. Statt dessen kann das Heizelemet auch spiral, - meander oder wellenförmig ausgebildet sein. Auch eine Zickzackform oder andere Formen sind möglich.

Der in der erfindungsgemäßen Inhalationsvorrichtung verwendet Behälter wird aus einer Aluminiumoxid-Keramik mittels Spritzgußverfahren hergestellt. Die verwendete Keramik hat eine Wärmeleitfähigkeit, die in der Größenordnung der Wärmeleitfähigkeit von Edelstahl liegt; erfindungsgemäß muß die Wärmeleitfähigkeit der verwendeten Keramik größer als 15 W/Km sein. Dies gewährt einen ausreichenden Wärmeübertrag an die zu erwärmende Flüssigkeit.

Bei der Herstellung des Keramikbehälters muß zudem eine Rißbildung vermieden werden, was durch die Gestaltung der Abmessungen erreicht wird. Ein Behälterdurchmesser von beispielsweise 34 mm bei einer Behälterhöhe von 36,5 mm und einer Wanddicke von typischerweise 2 mm erweist sich als geeignet. In der Regel sollte das Verhältnis von Behälterwanddicke zu Behälterhöhe zwischen 1:16 und 1:20 liegen, und das Verhältnis von Behälterwanddicke zu Behälterdurchmesser zwischen 1:15 und 1:19 liegen.

Zudem ist es vorteilhaft das Innere des Behälters, d.h. Innenseitenfläche 2a und Innenbodenfläche 3a, zu glasieren. Dadurch wird vermieden, daß die sich in dem Behälter befindenden Flüssigkeiten in die Wandfläche des Behälters eindringen, bzw. diese die Wandflächen verfärben. Dies ist bei der Verwendung in therapeutischen Inhalationsgeräten wichtig, da aufgrund der bestehenden Hygieneanforderungen eine gründliche Reinigung des Behälters notwendig ist, und Wirstoffrückstände in dem Behälter zu vermeiden sind.

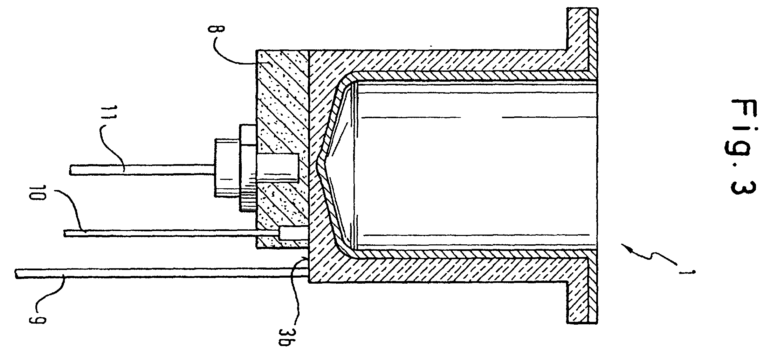

Im folgenden soll unter Bezugnahme auf Fig. 3 auf die Regelung des Heizelements eingegangen werden. Wie aus Fig. 3 ersichtlich, wird der Behälter 1 auf einer Isolierscheibe 8 angeordet. Die Isolierscheibe 8 kann auch auf der Aussenbodenfläche des Behälters aufgeklebt sein. Die Isolierscheibe besteht beispielsweise aus "Steatit" (Speckstein) oder einer anderen geeigneten Keramik. Eine Aluminiumoxid-Keramik kann ebenso verwendet werden. Die Isolierscheibe 8 hat im wesentlichen denselben Durchmesser wie der Boden 3 des Behälters, weist jedoch seitlich einen abgeflachten Bereich auf. Der Behälter ist bezüglich der Isolierscheibe 8 derart angeordnet, daß die Kontaktflächen 7a und 7b des Heizelements 6 über den abgeflachten Bereich der Isolierscheibe 8 überstehen. Somit werden die Anschlußleitungen 9a und 9b, über die das Heizelement mit Strom versorgt wird, an der Isolierscheibe 8 vorbeigeführt und können unmittelbar mit Kontaktflächen 7a und 7b in Kontakt treten. Die anderen Enden der Anschlußleitung 9a und 9b sind mit einer Energiequelle (nicht dargestellt) verbunden, die vorzugsweise eine 24V AC-Schutzkleinspannung bereitstellt. Durch die Verwendung eines Transformators wird hierbei die Netzspannung von 220V heruntertransformiert. Eine derartige Ausgestaltung ist insbesondere im Hinblick auf die Sicherheit des Patienten von Vorteil, da dieser die erfindungsgemäße Inhalationsvorrichtung während des Inhalationsvorgans in den Händen hält.

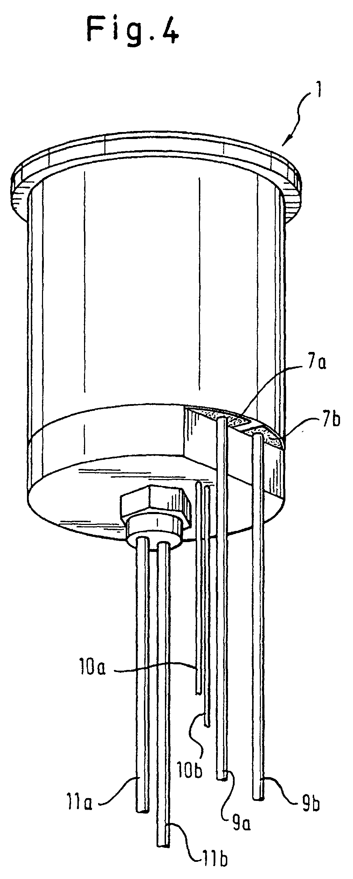

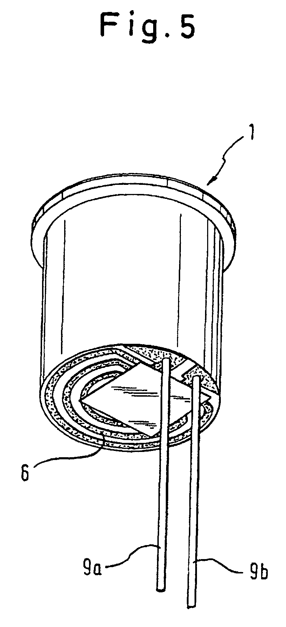

Ferner sind in die Isolierscheibe 8 die als Temperaturmeßfühler 10a und 10b dienenden NTC-Widerstände derart eingeführt, daß sie mit dem Behälterboden 3 in Kontakt treten, um unmittelbar die Temperatur des Behälterbodens messen zu können. Zudem ist ein Temperaturbegrenzer 11, umfassend Zuleitungen 11a und 11b, in die Isolierschicht integriert. Der Temperaturbegrenzer 11 bewirkt, daß die Stromversorgung über die Anschlußleitungen 9a und 9b automatisch abgeschaltet wird, wenn die Temperaturfühler (NTC-Widerstände) 10a und 10b einen maximalen Grenzwert erfassen. Wenn die von den NTC-Widerständen erfaßte Temperatur unter einen minimalen Grenzwert sinkt, schaltet der Temperaturbegrenzer 11 die Stromzufuhr an das Heizelement 6 automatisch ein. Die Anordnung von Temperaturbegrenzer 11, Teperaturfühler 10a und 10b und Stromleitungen 9a und 9b gehen aus Fig. 4 und 5 hervor.

## Patentansprüche

1. Inhalationsvorrichtung mit einem Behälter (1) zur Aufnahme einer Flüssigkeit und einem elektrischen Heizelement (6), das in wärmeleitendem Kontakt mit dem Behälter (1) steht, zum Erwärmen der in dem Behälter (1) aufgenommenen Flüssigkeit,
**dadurch gekennzeichnet, daß**
der Behälter (1) aus einer Keramik besteht, deren Wärmeleitfähigkeit λ > 15 W/Km ist, und das Heizelement (6) ein Dickschichtwiderstand ist, der auf der äußeren Oberfläche des Behälters (1) angeordnet ist.

2. Inhalationsvorrichtung nach Anspruch 1,
**dadurch gekennzeichnet, daß**
der Behälter (1) aus einer Aluminiumoxid-Keramik besteht.

3. Inhalationsvorrichtung nach Anspruch 1 und 2,
**dadurch gekennzeichnet, daß**
die Innenflächen des Behälters (2a, 3a) glasiert sind.

4. Inhalationsvorrichtung nach einem der vorangegangenen Ansprüche,
**dadurch gekennzeichnet, daß**
der Behälter (1) eine zur Mitte geneigte Innenbodenfläche (3a) aufweist.

5. Inhalationsvorrichtung nach einem der vorangegangenen Ansprüche,
**dadurch gekennzeichnet, daß**
die Aussenbodenfläche (3b) des Behälters plan geschliffen ist.

6. Inhalationsvorrichtung nach einem der vorangegangenen Ansprüche,
**dadurch gekennzeichnet, daß**
die Behälterwanddicke zur Behälterhöhe in einem Verhältnis von 1:16 bis 1:20 steht.

7. Inhalationsvorrichtung nach einem der vorangegangenen Ansprüche,
**dadurch gekennzeichnet, daß**
die Behälterwanddicke zum Behälterdurchmesser in einem Verhältnis von 1:15 bis 1:19 steht.

8. Inhalationsvorrichtung nach einem der vorangegangenen Ansprüche,
**dadurch gekennzeichnet, daß**
der Dickschichtwiderstand nur auf einer Aussenbodenfläche (3b) des Behälters angeordnet ist.

9. Inhalationsvorrichtung nach einem der vorangegangenen Ansprüche,
**dadurch gekennzeichnet, daß**
der Dickschichtwiderstand kreisförmig, spiralförmig, meanderförmig oder wellenförmig ist, oder eine Zickzackform aufweist.

10. Inhalationsvorrichtung nach einem der vorangegangenen Ansprüche,
**dadurch gekennzeichnet, daß**
der Mittelbereich (5) der Bodenfläche des Behälters nicht von dem Dickschichtwiderstand bedeckt wird.

11. Inhalationsvorrichtung nach einem der vorangegangenen Ansprüche,
**dadurch gekennzeichnet, daß**
der Dickschichtwiderstand einen Widerstand von ca. 12 Ω hat.

12. Inhalationsvorrichtung nach einem der vorangegangenen Ansprüche,
**dadurch gekennzeichnet, daß**
ein Isolierkörper vorgesehen ist, der auf die Aussenbodenfläche des Behälters geklebt wird und in dem eine Mehrzahl von Temperaturfühlern (10, 11) angeordnet ist.

13. Inhalationsvorrichtung nach Anspruch 12,
**dadurch gekennzeichnet, daß**
die in dem Isolierkörper angeordneten Temperaturfühler NTC-Widerstände (10) sind.

14. Inhalationsvorrichtung nach Anspruch 12,
**dadurch gekennzeichnet, daß** ein
Temperaturbegrenzer (11) vorgesehen ist.

15. Eine Inhalationsvorrichtung nach Anspruch 12,
**dadurch gekennzeichnet, daß**
der Isolierkörper (8) aus Keramik oder Steatit besteht.

16. Inhalationsvorrichtung nach einem der vorangegangenen Ansprüche,
**dadurch gekennzeichnet, daß**
das Heizelement (6) über die Anschlußleitungen (9a, 9b) mit einer 24V AC-Schutzkleinspannung verbunden ist.

## Claims

1. Inhaler with a container (1) for holding a liquid and an electric heating element (6) which is in heat-conducting contact with the container (1), for heating the liquid held in the container (1), **characterised in that** the container (1) is made of a ceramic whose thermal conductivity λ > 15 W/km, and the heating element (6) is a thick-film resistor which is arranged on the outer surface of the container (1).

2. Inhaler according to claim 1, **characterised in that** the container (1) is made of an aluminium oxide ceramic.

3. Inhaler according to claims 1 and 2, **characterised in that** the inner surfaces of the container (2a, 3a) are glazed.

4. Inhaler according to any of the preceding claims, **characterised in that** the container (1) comprises an inner bottom surface (3a) inclined towards the centre.

5. Inhaler according to any of the preceding claims, **characterised in that** the outer bottom surface (3b) of the container is surface-ground.

6. Inhaler according to any of the preceding claims, **characterised in that** the container wall thickness is in a ratio of 1:16 to 1:20 to the container height.

7. Inhaler according to any of the preceding claims, **characterised in that** the container wall thickness is in a ratio of 1:15 to 1:19 to the container diameter.

8. Inhaler according to any of the preceding claims, **characterised in that** the thick-film resistor is arranged only on an outer bottom surface (3b) of the container.

9. Inhaler according to any of the preceding claims, **characterised in that** the thick-film resistor is circular, spiral, meander-shaped or undulating or has a zigzag shape.

10. Inhaler according to any of the preceding claims, **characterised in that** the centre region (5) of the bottom surface of the container is not covered by the thick-film resistor.

11. Inhaler according to any of the preceding claims, **characterised in that** the thick-film resistor has a resistance of approx. 12 Ω.

12. Inhaler according to any of the preceding claims, **characterised in that** an insulating body is provided, which is glued to the outer bottom surface of the container and in which are arranged a plurality of temperature sensors (10, 11).

13. Inhaler according to claim 12, **characterised in that** the temperature sensors arranged in the insulating body are NTC resistors (10).

14. Inhaler according to claim 12, **characterised in that** a temperature limiter (11) is provided.

15. Inhaler according to claim 12, **characterised in that** the insulating body (8) is made of ceramic or steatite.

16. Inhaler according to any of the preceding claims, **characterised in that** the heating element (6) is connected by the connecting wires (9a, 9b) to a 24 V protective low a.c. voltage.

## Revendications

1. Dispositif pour inhalation comportant un récipient (1) devant recevoir un liquide et un élément chauffant (6) électrique, mis en contact conducteur de la chaleur avec le récipient (1) afin de chauffer le liquide se trouvant dans le récipient (1), **caractérisé en ce que** le récipient (1) est formé d'une céramique dont la conductivité thermique λ > 15 W/K.m et l'élément chauffant (6) est une résistance à couche épaisse, disposée sur la surface extérieure du récipient (1).

2. Dispositif d'inhalation selon la revendication 1, **caractérisé en ce que** le récipient (1) est formé d'une céramique à base d'oxyde d'aluminium.

3. Dispositif selon les revendications 1 et 2, **caractérisé en ce que** les faces intérieures du récipient (2a, 3a) sont émaillées.

4. Dispositif selon l'une des revendications précédentes, **caractérisé en ce que** le récipient (1) présente une face de fond intérieure (3a) inclinée vers le centre.

5. Dispositif selon l'une des revendications précédentes, **caractérisé en ce que** la face de fond extérieure (3b) du récipient est soumise à un usinage meulage l'ayant rendue plane.

6. Dispositif selon l'une des revendications précédentes, **caractérisé en ce que** le rapport, entre l'épaisseur de la paroi du récipient et la hauteur du récipient, est compris dans la plage allant de 1:16 à 1:20.

7. Dispositif selon l'une des revendications précédentes, **caractérisé en ce que** le rapport, entre l'épaisseur de paroi de récipient et le diamètre de récipient, est compris dans la plage allant de 1:15 à 1:19.

8. Dispositif selon l'une des revendications précédentes, **caractérisé en ce que** la résistance à couche épaisse n'est disposée que sur une face inférieure extérieure (3b) du récipient.

9. Dispositif selon l'une des revendications précédentes, **caractérisé en ce que** la résistance à couche épaisse est réalisée en forme de cercle, en forme de spirale, en forme de méandre ou en forme d'ondulation, ou bien présente une forme en zigzag.

10. Dispositif selon l'une des revendications précédentes, **caractérisé en ce que** la zone centrale (5) de la face de fond du récipient n'est pas couverte par la résistance à couche épaisse.

11. Dispositif selon l'une des revendications précédentes, **caractérisé en ce que** la résistance à couche épaisse présente une valeur de résistance électrique d'environ 12 Ω.

12. Dispositif selon l'une des revendications précédentes, **caractérisé en ce qu'**est prévu un corps isolant, collé sur la face de fond extérieur du récipient et dans lequel est disposée une pluralité de sondes de température (10, 11).

13. Dispositif selon la revendication 12, **caractérisé en ce que** les sondes de température disposées dans le corps isolant sont des résistances à caractéristique NTC (10).

14. Dispositif selon la revendication 12, **caractérisé en ce qu'**est prévu un limiteur de température (11).

15. Dispositif selon la revendication 12, **caractérisé en ce que** le corps isolant (8) est réalisé en céramique ou en stéatique.

16. Dispositif selon l'une des revendications précédentes, **caractérisé en ce que** l'élément chauffant (6) est relié à une basse tension de protection de courant alternatif AC d'une valeur de 24 Volts, par l'intermédiaire des lignes de raccordement (9a, 9b).
